# EUROPEAN PATENT APPLICATION

(11) **EP 3 422 223 A1**
(43) Date of publication of application: **02.01.2019**
(21) Application number: 17178948.0
(22) Date of filing: 30.06.2017
(51) Int. Cl.: G06F 19/00

(54) **GENERATING A PATIENT CARE PLAN**

(71) Applicant: Koninklijke Philips N.V., 5656 AE Eindhoven (NL)
(72) Inventor: WOLTHUIS, Jens, 5656 AE Eindhoven (NL); SOKORELI, Ioanna, 5656 AE Eindhoven (NL); VAN BERKEL, Joep, 5656 AE Eindhoven (NL); DE MASSARI, Daniele, 5656 AE Eindhoven (NL); ASIM, Muhammad, 5656 AE Eindhoven (NL); TESANOVIC, Aleksandra, 5656 AE Eindhoven (NL)
(74) Representative: de Haan, Poul Erik

(57) **Abstract**

There is provided a computer-implemented method (100) for generating a patient care plan. The method comprises acquiring (102) a plurality of single-condition care plans for a patient suffering from multiple medical conditions; enriching (104) at least one of the single-condition care plans; combining (106) the plurality of single-condition care plans into a multiple-condition care plan; and evaluating (108) a quality of the multiple-condition care plan relative to the plurality of single-condition care plans. A computer program product, an apparatus and a system are also disclosed.

## Description

### Technical Field of the Invention

The invention relates to patient care plans and, in particular, to generating a patient care plan for a patient having a plurality of medical conditions.

### Background to the Invention

The term multimorbidity refers to the presence of two or more chronic medical conditions in an individual. Multimorbid patients account for a large proportion of the users of health service facilities, and suffering from multiple chronic conditions can restrict normal activities associated with daily living.

Managing illnesses in multimorbid patients can be challenging as care for co-existing diseases is complex. The healthcare provided in respect of one of the diseases may overlap with, or even conflict with, the healthcare provided in respect of other diseases. Furthermore, there may be little coordination between healthcare professionals treating the individual conditions, meaning that the patient may be missing out on holistic support. Multimorbid patients who are not effectively treated may face an impaired quality of life, and an increased risk of further complications resulting from the inadequate care provided in respect of the combination of diseases.

Treating patients suffering from multiple chronic medical conditions can be costly, as such patients tend to require frequent hospital admissions and/or visits to specialists.

Individual illnesses (e.g. diseases) may be treated in accordance with a patient care plan, or clinical pathway. A clinical pathway sets out step-by-step, standardized guidance for a multi-disciplinary team to offer care for a specific disease (e.g. colon cancer) or situation (e.g. a patient entering an Emergency Department with chest pain). A patient care plan may be generated which is based on the clinical pathway, and is specific to the particular patient receiving the healthcare. While care plans are helpful for providing guidance in relation to single illnesses, for multimorbid patients, multiple care plans may be required, adding complexity to the care situation.

Therefore, it is desirable to have an improved mechanism for treating patients suffering from multiple chronic medical conditions, which seeks to address at least some of the problems discussed above.

### Summary of the Invention

According to a first aspect, the invention provides a computer-implemented method for generating a patient care plan. The method comprises acquiring a plurality of single-condition care plans for a patient suffering from multiple medical conditions; enriching at least one of the single-condition care plans; combining the plurality of single-condition care plans into a multiple-condition care plan; and evaluating a quality of the multiple-condition care plan relative to a quality of the plurality of single-condition care plans.

By combining the plurality of single-condition care plans into a single multiple-condition care plan, care utilisation can be reduced for the patient, thereby reducing healthcare expenditure. By further enriching the care plans, an optimised care plan may be generated for the patient.

Enriching a single-condition care plan may comprise supplementing the single-condition care plan with at least one of: i) information acquired from literature and/or clinical guidelines; and ii) details of additional tasks to be performed in relation to the patient. Enriching a care plan using information taken from clinical and/or non-clinical sources can lead to a more thorough care plan which, ultimately, may result in the patient receiving better and/or more effective healthcare for a lower cost to the healthcare provider.

In some embodiments, supplementing the single-condition care plan with information acquired from literature and/or clinical guidelines may comprise processing literature and/or clinical guidelines data using natural language processing to identify one or more tasks to be included in the single-condition care plan. Using natural language processing (NLP) techniques, additional information to be included in the care plans can be identified quickly, without the need for a user to trawl through documents manually to identify relevant information.

Each single-condition care plan may comprise a plurality of tasks to be performed in relation to the patient. The method may, in some embodiments, comprise identifying a particular task which appears in more than one of the plurality of single condition care plans; and combining the multiple instances of the particular task into a single task in the multiple-condition care plan. In this way, duplication of tasks can be avoided, helping to reduce care utilisation and save costs.

In some embodiments, the method may comprise identifying, for each of the multiple instances of the particular task, a timeframe within which the particular task is to be performed; and combining the multiple instances of the particular task into a single task in the multiple-condition care plan only if the particular task is to be performed in the multiple instances within the same timeframe.

In some embodiments, upon identifying that a particular task appears in more than one of the plurality of single-condition care plans, the method may comprise generating a signal to notify one or more users of the duplication of the particular task. The signal may be used to notify the caregivers who were scheduled to perform the plurality of tasks. In this way, all relevant caregivers can be made aware that the tasks will be combined, thereby reducing the workload for the caregivers.

Acquiring a single-condition care plan may, in some embodiments, comprise generating a single-condition care plan based on data from at least one of: a database of single-condition care plans for one or more reference subjects having a defined set of characteristics which are the same as, or similar to, characteristics of the patient, a medical record relating to the patient, and medical records relating to one or more reference subjects having a defined set of characteristics which are the same as, or similar to, characteristics of the patient.

In some embodiments, the step of evaluating may comprise obtaining a measure of effectiveness of treatment of the patient according to the multiple-condition care plan; obtaining a measure of effectiveness of treatment of one or more reference subjects according to the plurality of single-condition care plans, the one or more reference subjects having a defined set of characteristics which are the same as, or similar to, characteristics of the patient; comparing the measures of effectiveness of treatment of the patient and the one or more reference subjects; and upon determining that the measure of effectiveness of treatment of the patient according to the multiple-condition care plan is greater than the measure of effectiveness of treatment of one or more reference subjects according to the plurality of single-condition care plans, proposing replacement of the plurality of single-condition care plans with the multiple-condition care plan.

By evaluating the care plans in this way, it is possible to tell whether or not the changes made to the care plan (e.g. by enriching and combining the care plans) have helped to reduce care utilisation while maintaining the same (or improving) the level of care provided. If an improvement is evident, then the multiple-condition care plan may be used as a starting point for future similar patients. In this way, the care plan may be optimised for similar patients suffering from the same medical conditions, and/or patients who are similar in terms of health, socio-economic factors, environmental factors or behavioural factors.

The method may further comprise generating a request for approval, from a medical professional, to replace the plurality of single-condition care plans with the multiple-condition care plan.

The measure of effectiveness of treatment may comprise an improvement in health with a reduction in health care utilisation. Such an improvement may result in a corresponding reduction in healthcare costs.

According to a second aspect, the invention provides a computer program product comprising a non-transitory computer readable medium, the computer readable medium having computer readable code embodied therein, the computer readable code being configured such that, on execution by a suitable computer or processor, the computer or processor is caused to perform the method described above.

According to a third aspect, the invention provides an apparatus for generating a patient care plan. The apparatus comprises a memory comprising instruction data representing a set of instructions; and a processor. The processor is configured to communicate with the memory and to execute the set of instructions, wherein the set of instructions, when executed by the processor, cause the processor to perform the method described above.

According to a fourth aspect, the invention provides a system for generating a patient care plan, the system comprising an acquisition module for acquiring a plurality of single-condition care plans for a patient suffering from multiple medical conditions; an enrichment module for enriching at least one of the single-condition care plans; a combining module for combining the plurality of single-condition care plans into a multiple-condition care plan; and an evaluation module for evaluating a quality of the multiple-condition care plan relative to the plurality of single-condition care plans.

In some embodiments, the evaluation module may be configured to compare health outcomes of a patient treated according to the multiple-condition care plan with health outcomes of one or more reference subjects treated according to the plurality of single-condition care plans, the one or more reference subjects having a defined set of characteristics which are the same as, or similar to, characteristics of the patient.

The system may, in some embodiments, comprise a presentation module for presenting a proposal to replace the plurality of single condition care plans with the multiple-condition care plan, upon determining from the comparison that a measure of the health outcomes of the patient is greater than a measure of the health outcomes of the one or more reference subjects. The system may further comprise a user input module for receiving a user response to the presented proposal.

These and other aspects of the invention will be apparent from and elucidated with reference to the embodiments described hereinafter.

### Brief Description of the Drawings

For a better understanding of the invention, and to show more clearly how it may be carried into effect, reference will now be made, by way of example only, to the accompanying drawings, in which:
Figure 1 is a flowchart of an example of a method for generating a patient care plan, according to embodiments of the invention;
Figure 2 is a flowchart of a further example of a method for generating a patient care plan, according to embodiments of the invention;
Figure 3 is a flowchart of a further example of a method for generating a patient care plan, according to embodiments of the invention;
Figure 4 is a flowchart of a further example of a method for generating a patient care plan, according to embodiments of the invention;
Figure 5 is a flowchart of a further example of a method for generating a patient care plan, according to embodiments of the invention;
Figure 6 is a schematic illustration of an example computer-readable medium with a processor;
Figure 7 is a schematic illustration of an example of an apparatus, according to embodiments of the invention;
Figure 8 is a schematic illustration of an example of a system for generating a patient care plan, according to embodiments of the invention; and
Figure 9 is a schematic illustration of a further example of a system for generating a patient care plan, according to embodiments of the invention.

### Detailed Description of Embodiments

Embodiments of the invention disclosed herein provide an improved mechanism for providing healthcare to a patient having a plurality of medical conditions, particularly chronic medical conditions. A chronic medical condition is one which persists over a prolonged period of time, or which recurs repeatedly. Patient care plans can be established for individual medical conditions, based on clinical pathways which, for single medical conditions, may be well established. However, the invention disclosed herein provides methods, systems and apparatus for determining, or generating, a care plan suitable for a patient suffering from multiple medical conditions. Such co-existing conditions may be referred to as comorbid medical conditions.

For many common health problems, such as heart failure, chronic obstructive pulmonary disease and diabetes mellitus, there exist well-established healthcare guidelines (sometimes referred to as clinical pathways) which detail how a patient suffering from those health problems should be treated. From those guidelines, an individual care plan can be prepared for a disease for a particular patient. A care plan might include a series of tasks to be performed by one or more medical professionals. For example, a care plan might include a task requiring a clinician to carry out a clinical visit to the patient, a task requiring a nurse to take a blood sample from the patient, a task requiring a lab technician to analyse some test results from the patient, a task requiring a doctor to assess the patient, and so on.

For a patient suffering from multiple medical conditions, particularly multiple chronic medication conditions, each medication condition having its own care plan, there may be many tasks to be performed in respect of the patient, by many different medical professionals and, in some cases, multiple care plans may require the same task (e.g. take a blood sample) to be performed in respect of the patient by two or more different medical professionals. One or more of the different medical professionals may not be aware that another has been tasked with taking a blood sample from the same patient and, therefore, the patient could have multiple blood samples taken unnecessarily, causing a duplication in care utilisation by the medical professionals, worsening the patient's experience of the healthcare system, and an unnecessary increase in care costs.

Embodiments of the invention disclosed herein help to reduce the care utilisation for a patient suffering from multiple medical conditions, thereby reducing expenditure on care.

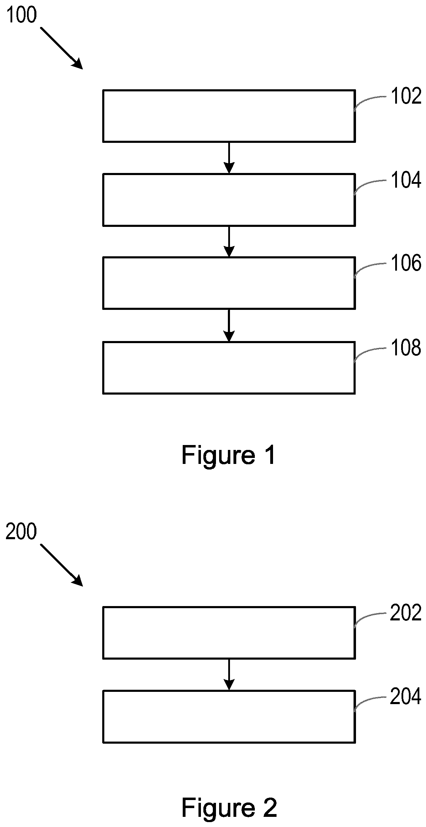

Referring to the drawings, Figure 1 is a flowchart of an example of a computer-implemented method 100 for generating a patient care plan. The method 100 may be performed by a processor of a computing device, or system. The method 100 comprises, at step 102, acquiring a plurality of single-condition care plans for a patient suffering from multiple medical conditions. Each care plan may relate to a respective one of the medical conditions from which the patient is suffering. For example, the patient may be receiving care to treat diabetes mellitus according to a diabetes care plan while, at the same time, receiving care to treat a chronic pulmonary disease according to a pulmonary disease care plan. Each care plan may comprise a list of tasks to be performed by various medical professionals. The care plans associated with the patient may be stored in a storage medium (e.g. in a database) of a computing device or computer system, for example in a cloud computing environment. The step of acquiring 102 the care plans may, for example, involve downloading the care plans from an external storage device. In some embodiments, the care plans may be acquired by a user (e.g. medical professional) inputting the care plan into a computer system, for example by uploading the care plan, or entering the care plan manually, using a user input interface.

A single-condition care plan may be generated in a variety of ways. In some examples, a single-condition care plan for a condition may be based at least in part on clinical guidelines or established clinical pathways relevant to the condition. For example, in the UK, guidelines provided by the National Institute for Health and Care Excellent (NICE) may be included in a care plan, or used to form the care plan. In some examples, a single-condition care plan may be generated based at least in part on data included in healthcare cost information, such as information regarding a healthcare insurance claim. Such so-called "healthcare claim data" may include details of multiple diagnoses of a patient, procedures performed in respect of the patient, and amount paid for treatment administered, and information about the patient. In some examples, data from health records (e.g. an electronic health record (EHR)) of a subject (e.g. a previous patient) or group of subjects who are similar (e.g. medically similar or similar in other respects) to the patient may be taken into account to design a care plan. In the context of this invention, a subject who is "medically similar" to a patient is considered to have characteristics which are similar to or the same as those of the patient. In the context of this invention, a subject who has characteristics which are "similar" to characteristics of the patient is considered to be similar enough from a medical point of view that the same treatment for a medical condition might be expected to be successful for both the subject and the patient. In some embodiments, a set of characteristics may be considered to be similar to another set of characteristics if they statistically match in their likeness. In some embodiments, a set of characteristics may be the same as another set of characteristics if they are substantially the same. Such characteristics may, for example, include gender, age, medical history, current illnesses, and the like. In some examples, details of the patient may be taken into account when developing a care plan. For example, details included in the patient's electronic health record or medical history, and/or details from other sources such as healthcare purchasing data, healthcare claims data or demographic data may be taken into account. Such records may provide information regarding allergies or phobias which might affect which tasks can be included in the care plan. Thus, the step of acquiring 102 a single-condition care plan may comprise generating a single-condition care plan based on data from at least one of: a database of single-condition care plans for one or more reference subjects having a defined set of characteristics which are the same as, or similar to, characteristics of the patient, a medical record relating to the patient, and medical records relating to one or more reference subjects having a defined set of characteristics which are the same as, or similar to, characteristics of the patient. Single-condition care plans relating the reference subjects stored in a database may be care plans which have been defined by health organisations (e.g. NICE) based on clinical guidelines.

At step 104, the method 100 comprises enriching at least one of the single-condition care plans. The step of enriching a care plan is intended to enhance, improve or modify the care plan in some way in an attempt to improve the level of care provided to a patient being treated in accordance with the care plan. For example, the care plan may be cross-checked with another care plan to identify any tasks that may have been omitted, but which may improve the care plan. In some examples, the care plan may be enriched by including one or more tasks, or by reordering or otherwise modifying one or more tasks, based on a recommendation, opinion or advice from a medical professional, such as a medical professional familiar with the care plan, or a medical professional who has experience working in the field of the medical condition to which the care plan relates.

In some embodiments, enriching a single-condition care plan may comprises supplementing the single-condition care plan with at least one of: i) information acquired from literature and/or clinical guidelines; and ii) details of additional tasks to be performed in relation to the patient. Supplementing the single-condition care plan with information acquired from literature and/or clinical guidelines may comprise processing literature and/or clinical guidelines data using natural language processing (NLP) to identify one or more tasks to be included in the single-condition care plan. For example, clinical guidelines, clinical pathways, sources of literature (such as textbooks, academic papers, journals and the like), and/or comments, advice or papers written by other clinicians and experts may be analysed using NLP techniques. NLP may be used to identify relevant passages in the text of such sources, relating to the condition to which the care plan relates, or to an optimal way of treating the condition. Any additional tasks that might be identified from the NLP may, if considered helpful, be included in the care plan to enrich the plan and, thereby, to enrich the care provided to the patient. In some embodiments, aspects of the enriching (e.g. the NLP techniques) may be performed by a processor other than the processor on which the method 100 is performed.

The method 100 comprises, at step 106, combining the plurality of single-condition care plans into a multiple-condition care plan. In the simplest sense, combining the plurality of single-condition care plans may comprise conflating the tasks in the care plans into a single combined list. For example, if a first single-condition care plan for a patient included tasks A, B and C, and a second single-condition care plan for the patient included tasks D, E and F, then combining the first and second single-condition care plans might generate a multiple-condition care plan which includes tasks A, B, C, D, E and F.

The method 100 comprises, at step 108, evaluating a quality of the multiple-condition care plan relative to the plurality of single-condition care plans. In other words, the quality of the multiple-condition care plan and the quality of the single-condition care plans are evaluated, for example by performing a comparison. In some embodiments, the relative quality of the multiple-condition care plan and the single-condition care plans may be evaluated by comparing health outcomes of patient treated by the multiple-condition care plan with health outcomes of patients treated by the single-condition care plans. By evaluating the quality of the care plans, an optimised care plan can be developed. In other embodiments, other metrics (e.g. healthcare costs) may be used to evaluate the quality of the multiple-condition care plan relative to the single-condition care plans. Changes in the care plan (e.g. from the enriching and combining steps) which result in an improved care plan can be accepted and implemented into future care plans, while changes which do not result in an improved care plan can be reversed, or removed from the care plans. In the context of this invention, an improved care plan may be considered to be one which results in a reduction in care utilisation in respect of a patient, but which results in the same or better health outcomes for the patient.

Figure 2 shows a flowchart of a further example of a method 200 for generating a patient care plan. The method 200 may include one or more steps from the method 100 described above. The method 200 comprises, at step 202, identifying a particular task which appears in more than one of the plurality of single condition care plans. At step 204, the method 200 comprises combining the multiple instances of the particular task into a single task in the multiple-condition care plan. For example, if first and second single-condition care plans both include a task requiring a nurse to take a blood sample then, to prevent a duplication in work (i.e. to avoid two nurses taking separate blood samples from the patient, or one nurse taking two blood samples from the patient), the duplicated task may be combined into a single task. For example, one of the duplicated tasks may be deleted.

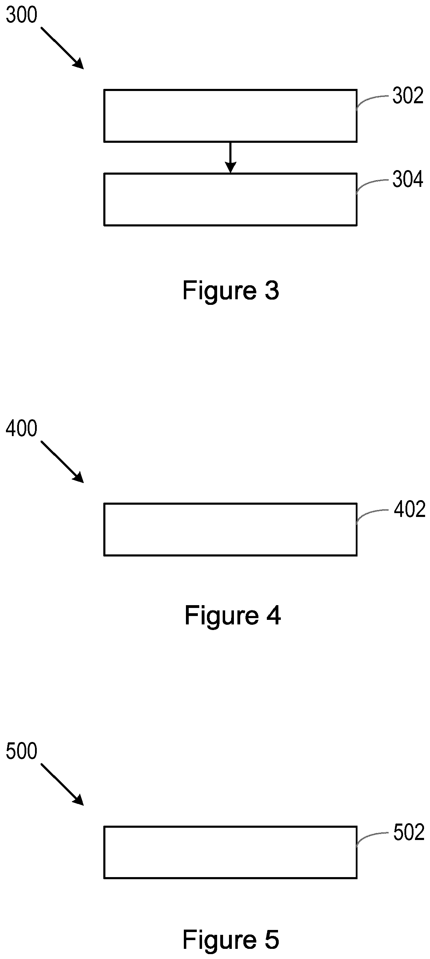

Figure 3 shows a flowchart of a further example of a method 300 for generating a patient care plan. The method 300 may include one or more steps from the methods 100, 200 described above. The method 300 comprises, at step 302, identifying, for each of the multiple instances of the particular task, a timeframe within which the particular task is to be performed. At step 304, the method 300 may comprise combining the multiple instances of the particular task into a single task in the multiple-condition care plan only if the particular task is to be performed in the multiple instances within the same timeframe. For example, the method 300 may identify (step 302) that both of the single-condition care plans require that a blood sample is taken from the patient immediately before lunch is served, or between 11:30am and 12:00pm, for example. In this case, the multiple instances of the task to take a blood sample would be combined (step 304) into a single task, so that the work is not duplicated unnecessarily. In some embodiments, a timeframe may be defined as a specific duration of time (e.g. between 9:00am and 9:10am) while, in other embodiments, a broader time range may be defined (e.g. during the morning).

Since care plans may include tasks to be performed by multiple care-givers, or medical professionals, each of whom could be located in a different medical facility, it is important that a relevant medical professional is notified in the event that a duplication of tasks in a plurality of single-condition care plans is identified. Figure 4 shows a flowchart of a further example of a method 400 for generating a patient care plan. The method 400 may include one or more steps from the methods 100, 200, 300 described above. The method 400 comprises, at step 402, generating a signal to notify one or more users of the duplication of the particular task, upon identifying that a particular task appears in more than one of the plurality of single-condition care plans. Such a notification signal may comprise a signal which is delivered to a mobile communication device (e.g. a pager or mobile telephone) of a relevant medical professional, or a signal to cause the duplication to be presented on a display screen. In some embodiments, if a duplication of tasks is identified in multiple single-condition care plans, then the medical professional responsible for each single-condition care plan may be notified. In this way, all of the medical professions who should know about the task can be informed. Furthermore, if a first medical professional is no longer required to perform a particular task in a patient's care plan (e.g. because it is to be performed by a second medical professional), then the first medical professional may utilise their spare time performing another task.

According to some embodiments of the invention, the step of evaluating 108 a quality of the multiple-condition care plan relative to a quality of the plurality of single-condition care plans comprises obtaining a measure of effectiveness of treatment of the patient according to the multiple-condition care plan. The measure of effectiveness may comprise a numerical value (e.g. a percentage) which serves as an indication of how effective treatment is according to the care plan. In some embodiments, some other metric may be used as the measure of effectiveness. In some embodiments, the measure of effectiveness of treatment may comprise an improvement in health with a reduction in health care utilisation.

The evaluating step 108 may further comprise obtaining a measure of effectiveness of treatment of one or more reference subjects according to the plurality of single-condition care plans, the one or more reference subjects having a defined set of characteristics which are the same as, or similar to, characteristics of the patient. The measure of effectiveness of treatment of one or more reference subjects is, preferably, equivalent to, or based on the same factors as, the measure of effectiveness of treatment of the patient. To ensure an accurate useful comparison, the one or more reference subjects have at least some characteristics the same as, or similar to, those of the patient. In this way, the reference subjects can be considered to be similar (e.g. medically similar) to the patient and, as such, treatments may be considered to be comparable.

The evaluating step 108 may further comprise comparing the measures of effectiveness of treatment of the patient and the one or more reference subjects. In an example where the measure of effectiveness of treatment is a numerical value, then the step of comparing may comprise comparing the numerical values to determine which value is largest. The measure of effectiveness having the largest value may be determined to be the more effective. In other examples, a smaller numerical value may indicate a better measure of effectiveness. In some embodiments, where the measure of effectiveness of treatment is not a numerical value, then the measures may be compared in some other way.

Thus, the method may comprise determining which of the measures of effectiveness of treatment is greater, or which treatment is more effective.

Upon determining that the measure of effectiveness of treatment of the patient according to the multiple-condition care plan is greater than the measure of effectiveness of treatment of one or more reference subjects according to the plurality of single-condition care plans, the evaluating step 108 may further comprise proposing replacement of the plurality of single-condition care plans with the multiple-condition care plan. In some embodiments, the proposal may comprise a proposal to replace the plurality of single-condition care plans with the multiple-condition care plan for future patients (i.e. patients for whom treatment is yet to commence) suffering from the same medical conditions. In some embodiments, the proposal may comprise a proposal to replace the plurality of single-condition care plans with the multiple-condition care plan for future patients having a defined set of characteristics which are the same as, or similar to, characteristics of the patient. In other words, it may be proposed to replace the existing single-condition care plans with a single multiple-condition care plan only for those patients who are considered to be medically similar to the patient. If the more effective and/or efficient multiple-condition care plan is adopted as the standard care plan for future patients (particularly similar patients) suffering from the same or similar medical conditions, then treatment of those future patients is likely to be more effective. In some embodiments, the step of proposing replacement of the care plan may be performed separately to the evaluating step 108.

In some embodiments, the multiple-condition care plan may be used as the standard care plan for patients suffering from the same conditions as the patient, rather than the plurality of single-condition care plans. The multiple-condition care plan may then be enriched in a manner similar to that described above in the method 100, so as to further improve the care plan and improve the effectiveness of treatment according to the care plan. In this way, the multiple-condition care plan for particular combinations of medical conditions may be repeatedly improved and optimised for patients with particular characteristics. By optimising the care plans, levels of care utilisation may decrease, and health outcomes of patients treated according to the care plans remain the same, or improve. This may result in a decrease in expenditure in the care required to achieve the same overall effect for a patient.

Figure 5 shows a flowchart of a further example of a method 500 for generating a patient care plan. The method 500 may include one or more steps from the methods 100, 200, 300, 400 described above. The method 500 comprises, at step 502, generating a request for approval, from a medical professional, to replace the plurality of single-condition care plans with the multiple-condition care plan. Approval to make a change to the care plan, such as to replace multiple single-condition care plans with a multiple-condition care plan, may be required from a medical professional. In some embodiments, approval of a medical profession of a defined minimum level of authority may be required for a change to be implemented. The request for approval may be displayed on a display screen of a computing device for the appropriate medical professional to view, for example when he or she is reviewing an electronic health record of the patient. In some embodiments, the approval request may be delivered to a mobile device of the medical professional. In some embodiments, it may be intended to replace the single-condition care plans with the multiple-condition care plan so that the multiple-condition care plan is used for future patients that are similar to (i.e. have characteristics similar to) the patient for whom the multiple-condition care plan has been generated. The single-condition care plans may still be used for patients who have a single condition.

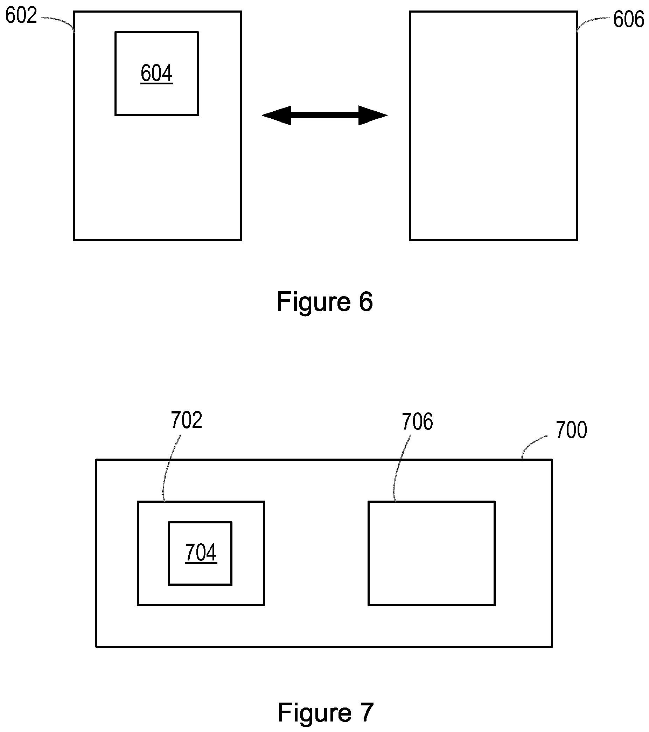

A further aspect of the invention relates to a computer program product comprising a computer-readable medium. Figure 6 is a schematic illustration of an example computer-readable medium 602 with a processor 606. The computer-readable medium 602 includes computer readable code 604 embodied therein, the computer readable code being configured such that, on execution by a suitable computer or processor 604, the computer or processor is caused to perform the method 100-500 described above.

A further aspect of the invention relates to an apparatus for generating a patient care plan. Figure 7 is a schematic illustration of an example of an apparatus 700, according to embodiments of the invention. The apparatus 700 comprises a memory 702 comprising instruction data representing a set of instructions 704. The apparatus 700 further comprises a processor 706 configured to communicate with the memory 702 and to execute the set of instructions, wherein the set of instructions 704, when executed by the processor, cause the processor to perform the method 100-500 described above.

The processor 706 can comprise one or more processors, processing units, multi-core processors or modules that are configured or programmed to control the apparatus 700 in the manner described herein. In particular implementations, the processor 706 can comprise a plurality of software and/or hardware modules that are each configured to perform, or are for performing, individual or multiple steps of the method described herein.

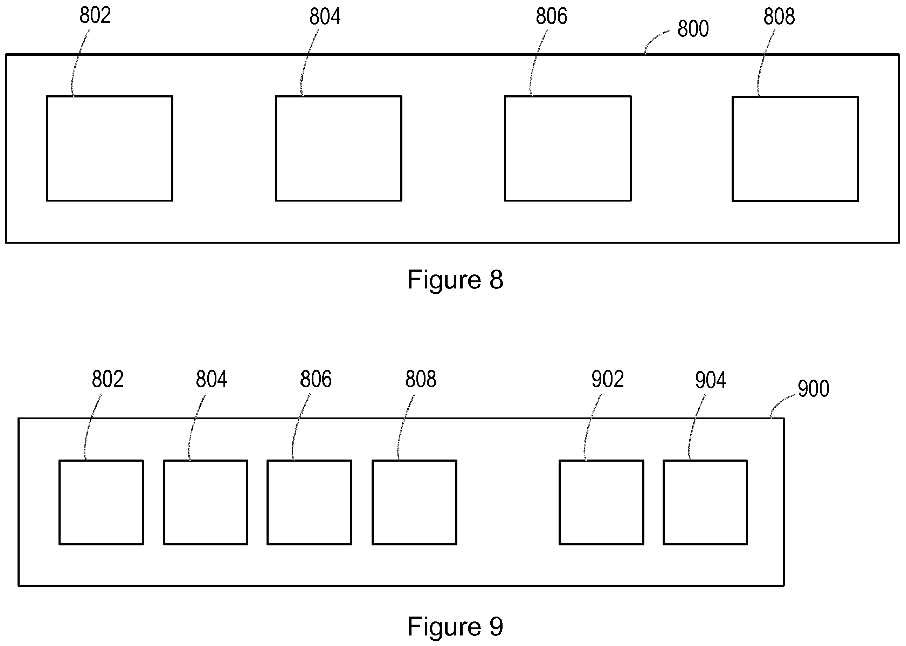

A further aspect of the invention relates to a system for generating a patient care plan. Figure 8 is a schematic illustration of an example of a system 800, according to embodiments of the invention. The system 800 comprises an acquisition module 802 for acquiring a plurality of single-condition care plans for a patient suffering from multiple medical conditions; an enrichment module 804 for enriching at least one of the single-condition care plans; a combining module 806 for combining the plurality of single-condition care plans into a multiple-condition care plan; and an evaluation module 808 for evaluating a quality of the multiple-condition care plan relative to the plurality of single-condition care plans. In general, the system 800 may comprise modules configured to perform the method 100-500 described above.

The system 800 may be implemented as a collection of computer software modules (802, 804, 806, 808) configured to operate on a computer system, such as a desktop computer, a laptop computer, a tablet computer or a smartphone. In some embodiments, the system 800 may be implemented over multiple computing devices or servers, and may be implemented partly or fully in a cloud computing environment.

The evaluation module 808 may, in some embodiments, be configured to compare health outcomes of a patient treated according to the multiple-condition care plan with health outcomes of one or more reference subjects treated according to the plurality of single-condition care plans, the one or more reference subjects having a defined set of characteristics which are the same as, or similar to, characteristics of the patient. The comparison, according to some embodiments, may be the same as, or similar to, the comparison performed as part of the evaluation step 108, described above. The health outcomes may be compared by comparing equivalent metrics of the health of the patient and the reference subjects following treatment according to the respective care plans.

Figure 9 is a schematic illustration of a further example of a system 900, according to embodiments of the invention. The system 900 may include one or more components (e.g. modules) of the system 800. The system 900 may further comprise a presentation module 902 for presenting a proposal to replace the plurality of single condition care plans with the multiple-condition care plan, upon determining from the comparison that a measure of the health outcomes of the patient is greater than a measure of the health outcomes of the one or more reference subjects. In some embodiments, the system 900 may further comprise a user input module 904 for receiving a user response to the presented proposal. The user response may, for example, be an approval or rejection of the proposal to replace the plurality of single condition care plans with the multiple-condition care plan.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for generating a patient care plan, the method comprising:
acquiring (102) a plurality of single-condition care plans for a patient suffering from multiple medical conditions;
enriching (104) at least one of the single-condition care plans;
combining (106) the plurality of single-condition care plans into a multiple-condition care plan; and
evaluating (108) a quality of the multiple-condition care plan relative to a quality of the plurality of single-condition care plans.

2. The method of claim 1, wherein enriching (104) a single-condition care plan comprises supplementing the single-condition care plan with at least one of: i) information acquired from literature and/or clinical guidelines; and ii) details of additional tasks to be performed in relation to the patient.

3. The method of claim 2, wherein supplementing the single-condition care plan with information acquired from literature and/or clinical guidelines comprises:
processing literature and/or clinical guidelines data using natural language processing to identify one or more tasks to be included in the single-condition care plan.

4. The method of any of the preceding claims, further comprising:
identifying (202) a particular task which appears in more than one of the plurality of single-condition care plans; and
combining (204) the multiple instances of the particular task into a single task in the multiple-condition care plan.

5. The method of claim 4, further comprising:
identifying (302), for each of the multiple instances of the particular task, a timeframe within which the particular task is to be performed; and
combining (304) the multiple instances of the particular task into a single task in the multiple-condition care plan only if the particular task is to be performed in the multiple instances within the same timeframe.

6. The method of claim 4 or claim 5, further comprising:
upon identifying that a particular task appears in more than one of the plurality of single-condition care plans, generating (402) a signal to notify one or more users of the duplication of the particular task.

7. The method of any of the preceding claims, wherein acquiring a single-condition care plan comprises:
generating a single-condition care plan based on data from at least one of: a database of single-condition care plans for one or more reference subjects having a defined set of characteristics which are the same as, or similar to, characteristics of the patient, a medical record relating to the patient, and medical records relating to one or more reference subjects having a defined set of characteristics which are the same as, or similar to, characteristics of the patient.

8. The method of any of the preceding claims, wherein the step of evaluating comprises:
obtaining a measure of effectiveness of treatment of the patient according to the multiple-condition care plan;
obtaining a measure of effectiveness of treatment of one or more reference subjects according to the plurality of single-condition care plans, the one or more reference subjects having a defined set of characteristics which are the same as, or similar to, characteristics of the patient;
comparing the measures of effectiveness of treatment of the patient and the one or more reference subjects; and
upon determining that the measure of effectiveness of treatment of the patient according to the multiple-condition care plan is greater than the measure of effectiveness of treatment of one or more reference subjects according to the plurality of single-condition care plans, proposing replacement of the plurality of single-condition care plans with the multiple-condition care plan.

9. The method of claim 8, further comprising:
generating (502) a request for approval, from a medical professional, to replace the plurality of single-condition care plans with the multiple-condition care plan.

10. The method of claim 8 or claim 9, wherein the measure of effectiveness of treatment comprises an improvement in health with a reduction in health care utilisation.

11. A computer program product comprising a non-transitory computer readable medium, the computer readable medium (602) having computer readable code (604) embodied therein, the computer readable code being configured such that, on execution by a suitable computer or processor (606), the computer or processor is caused to perform the method (100-500) of any of the preceding claims.

12. An apparatus (700) for generating a patient care plan, the apparatus comprising:
a memory (702) comprising instruction data representing a set of instructions (704); and
a processor (706) configured to communicate with the memory and to execute the set of instructions, wherein the set of instructions, when executed by the processor, cause the processor to perform the method (100-500) of any of claims 1 to 10.

13. A system (800) for generating a patient care plan, the system comprising:
an acquisition module (802) for acquiring a plurality of single-condition care plans for a patient suffering from multiple medical conditions;
an enrichment module (804) for enriching at least one of the single-condition care plans;
a combining module (806) for combining the plurality of single-condition care plans into a multiple-condition care plan; and
an evaluation module (808) for evaluating a quality of the multiple-condition care plan relative to the plurality of single-condition care plans.

14. The system (800) of claim 13, wherein the evaluation module (808) is configured to compare health outcomes of a patient treated according to the multiple-condition care plan with health outcomes of one or more reference subjects treated according to the plurality of single-condition care plans, the one or more reference subjects having a defined set of characteristics which are the same as, or similar to, characteristics of the patient.

15. The system (800, 900) of claim 14, further comprising:
a presentation module (902) for presenting a proposal to replace the plurality of single-condition care plans with the multiple-condition care plan, upon determining from the comparison that a measure of the health outcomes of the patient is greater than a measure of the health outcomes of the one or more reference subjects; and
a user input module (904) for receiving a user response to the presented proposal.
